# FASCICULE DE BREVET EUROPEEN

(11) **EP 2 044 189 B1**
(45) Date de publication et mention de la délivrance du brevet: **12.02.2014**
(21) Numéro de dépôt: 07803874.2
(22) Date de dépôt: 16.07.2007
(51) Int. Cl.: C11D 3/04, C11D 17/00, C11D 3/22, C11D 3/02, C11D 3/00, A61K 9/20, A61L 2/232

(54) **TABLETTE MULTICOUCHE À COUCHES DE FRAGMENTATION**
MEHRSCHICHTIGE TABLETTE MIT ZERFALLSCHICHT
MULTILAYER TABLET HAVING A DISINTEGRATION LAYER

(30) Priorité: 18.07.2006 FR 0606552
(43) Date de publication de la demande: 08.04.2009
(73) Titulaire: Eurotab, 42170 Saint Just Saint Rambert (FR)
(72) Inventeur: RUBINSTENN, Gilles, 75005 Paris (FR); JEANTET, Christine, 42160 Andrezieux Boutheon (FR); MATHIEU, Catherine, 43000 Le Puy en Velay (FR)
(74) Mandataire: Brédeville, Odile Marie
(86) Numéro de dépôt international: PCT/FR2007/001214
(87) Numéro de publication internationale: WO 2008/009804

(56) Documents cités:
- EP-A- 1 352 949
- FR-A1- 2 559 499
- GB-A- 1 346 609

## Description

La présente invention porte sur une tablette solide à plusieurs couches, apte à se déliter dans un liquide.

Des compositions solides, sous forme de tablettes ou de pastilles aptes à se déliter dans un liquide sont connues, par exemple dans le domaine de la détergence, du traitement des eaux, des engrais, etc.... En effet, la présentation d'un produit sous forme solide, ou compacte, présente des avantages : le produit est facile à manipuler, à stocker, à doser. Ainsi, on préférera souvent préparer une composition solide pour stocker un agent actif appelé à être libéré dans un liquide, par exemple de l'eau, pour fournir une solution active, par exemple apte à nettoyer ou désinfecter, ou encore apte à fertiliser des plantations ou à traiter une eau non potable. Ces compositions comprennent généralement un support capable de se dissoudre dans ledit liquide.

Les pastilles ou tablettes sous forme solide sont généralement préparées à partir de grains des différents constituants fournis sous forme de poudres que l'on compacte : par délitement, on entend au sens de la présente demande, une séparation grossière des grains, l'étape de dissolution proprement dite pouvant intervenir simultanément ou ultérieurement.

On comprend aisément qu'une propriété importante de ces compositions solides est leur aptitude à se déliter rapidement ou tout du moins d'une façon contrôlable par le formulateur.

Pour augmenter la vitesse de délitement de ces compositions, on leur a adjoint des agents d'aide au délitement : ces agents sont généralement appelés agents d'éclatement. Ces agents agissent par gonflement, déformation plastique, effet mèche, lorsqu'ils entrent au contact de l'eau ou du liquide dans lequel on cherche à dissoudre la tablette.

Malgré la présence de ces agents d'éclatement, il arrive que le temps de délitement de ces pastilles ou tablettes soit encore trop long pour l'utilisation souhaitée.

Par ailleurs, les agents d'éclatement occupent dans la tablette ou la pastille un volume, de fait non disponible pour les ingrédients actifs de la formule. Ces agents d'éclatement, en dehors de leur aptitude à aider au délitement de la tablette, présentent en général un intérêt fonctionnel limité, voire nul, en ce qui concerne l'application souhaitée pour la tablette : ils ne présentent généralement pas de propriétés détergentes, biocides, ou autres. Ces agents d'éclatement peuvent également constituer des rejets éventuellement nuisibles à l'environnement et peuvent induire des problèmes de colmatage de filtre pour certaines applications lorsqu'ils sont de nature insoluble.

Le document GB 1 346 609 décrit des tablettes tricouches comprenant une couche intermédiaire désintégrable disposée entre une première couche et une deuxième couche externes. Toutefois, les tablettes tricouches de GB 1 346 609 sont entièrement recouvertes d'un revêtement à l'exception d'une face de la première couche externe qui reste exposée. Ainsi, au cours du délitement d'une tablette de GB 1 346 609, la première couche externe se délite, puis la couche intermédiaire, puis la deuxième couche externe. La couche intermédiaire ne se délite donc pas avant la première couche externe. Dans GB 1 346 609, la couche intermédiaire est présente fournir un séquençage dans la libération de l'actif et donc un ralentissement par rapport à ce qui serait obtenu en cas d'absence de ces couches.

Ainsi, il subsiste le besoin de fournir des compositions solides, comme des pastilles ou des tablettes, pour lesquelles le temps de délitement soit optimisé, en particulier réduit, tout en limitant l'utilisation d'agents d'éclatement.

La demanderesse a découvert de façon surprenante que contrairement à ce qui est suggéré par l'état de l'art formulatoire et soutenu par les notices techniques des agents d'éclatement disponibles sur le marché, ceux-ci se révèlent plus efficaces lorsqu'ils sont concentrés dans une couche de fragmentation, toute chose égale par ailleurs, que lorsqu'ils sont dilués dans le corps du compact dont ils ont vocation à accélérer le délitement.

La présente invention donc vise à remédier à ce besoin en proposant des tablettes dont le taux global en agent d'éclatement reste raisonnable tout en permettant un temps de délitement réduit par rapport aux tablettes ou pastilles de l'art antérieur.

La présente invention porte sur une tablette solide multicouche, destinée à se déliter dans un liquide pour libérer dans ledit liquide un agent actif, comprenant au moins n couches de fragmentation, n étant un nombre entier au moins égal à 1, et au moins (n + 1) couches de produit, lesdites (n + 1) couches de produit étant de compositions identiques, chaque couche de produit comprenant au moins ledit agent actif, chaque couche de fragmentation étant disposée entre deux couches de produit et présentant un délitement significativement plus rapide que celui desdites couches de produit,
- chaque couche de fragmentation comprenant au moins un agent d'éclatement, ledit agent d'éclatement étant présent dans ladite couche de fragmentation à une teneur supérieure ou égale à 25% en poids, par rapport au poids de la dite couche de fragmentation, caractérisée en ce que le taux du poids total de l'ensemble des couches de fragmentation rapporté au poids total de l'ensemble des couches de produit est au plus égal à 33% l'agent d'éclatement est présent dans la tablette à une teneur inférieure ou égale à 15% en poids par rapport au poids total de la tablette, et au moins une couche de fragmentation comprend un sel soluble.

Grâce à la disposition spécifique des différentes couches de la tablette selon l'invention, celle-ci se délite en un temps accéléré par rapport aux tablettes de l'art antérieur. En particulier, la tablette selon l'invention se délite de façon significativement plus rapide qu'une tablette monocouche comprenant le même taux global d'agent d'éclatement.

Dans la présente demande, on entend, par tablette un produit compact en trois dimensions. Ainsi, le terme « tablette » comprend les termes comprimé, pastille, galet, etc... Cette pastille peut présenter des faces supérieure et inférieure plates ou bombées, concaves ou convexes. Cette tablette peut être indifféremment de forme générale ronde, ovale, carrée, rectangulaire, octogonale, polygonale. De préférence, la tablette selon l'invention est généralement de forme générale parallélépipédique, dont les sommets et les arrêtes sont plus ou moins adoucis voir tronqués.

La tablette selon l'invention comprend au moins deux sortes de couches : des couches dites couches de produit et au moins une couche dite de fragmentation, disposée entre deux couches de produit. Ainsi, la tablette selon l'invention comprend au moins trois couches, deux couches de produit et une couche de fragmentation. De façon générale, la tablette selon l'invention comprend n couches de fragmentation, n étant un nombre entier au moins égal à 1, et (n + 1) couches de produit. Chaque couche de fragmentation est disposée entre deux couches de produit et sépare ces deux couches de produit.

Dans une forme de réalisation de l'invention, la tablette comprend deux couches de fragmentation et trois couches de produit, disposées en alternance, de la façon suivante : couche produit/couche de fragmentation/couche produit/couche de fragmentation/couche produit.

Dans une autre forme de réalisation de l'invention, la tablette comprend trois couches de fragmentation et quatre couches de produit.

Dans une forme de réalisation de l'invention, la tablette comprend au moins deux couches de fragmentation, de préférence au moins trois couches de fragmentation, et de préférence encore au moins quatre couches de fragmentation.

Les couches de produit comprennent au moins un agent actif. Cet agent actif peut varier en fonction de l'application envisagée pour la tablette. De préférence, cet agent actif est choisi parmi les agents détergents, les agents désinfectants, les agents de blanchiment, les agents fertilisants, les pesticides, les agents coagulants, les agents floculants, les agents présentant un intérêt cosmétique, nutritionnel ou nutraceutique, les catalyseurs chimiques ou biotechnologiques, les teintures, et leurs mélanges.

Comme agents détergents, on peut citer les tensioactifs cationiques, les tensioactifs anioniques, les tensioactifs non ioniques et leurs mélanges.

Comme agents désinfectants, on peut citer tout dérivé de chlore connu pour libérer du chlore actif, comme par exemple le sel de sodium de N-chloro-4-méthylbenzène sulfonamide sous forme anhydre ou dihydratée, le sel de sodium de 1,3-dichloro-s-triazine-2,4,6 trione sous forme anhydre ou dihydratée, et leurs mélanges.

Comme agents de blanchiment, on peut citer le perborate de sodium mono et tétra hydraté, le carbonate de sodium péroxyhydraté, le monopérisophalate de magnésium xH₂O, le monopersulfate de potassium l'acide trichloroisocyanurique, les sels de sodium ou potassium de dichloroisocyanurates hydratés ou anhydres, et leurs mélanges.

Comme agents fertilisants, on peut citer particulièrement l'acide phosphorique ou les oligomères du phosphate de sodium.

Comme agent coagulant, on peut citer les sels de cations divalents et trivalent, en particulier, les sels de l'aluminium et du fer de degré d'oxydation III.

Comme agent floculant, on peut citer les polymères de l'acrylamide tels que le « FLOPAM AM934 », commercialisé par la société SNF.

Comme agent présentant un intérêt cosmétique, on peut citer des dérivés hydrophiles de la vitamine E, la vitamine C, le rétinol, les dérivés d'hydroxystylbènes et plus précisément le resvératrol, les caroténoides, le lycopène, les isoflavonoïdes et plus précisément les isoflavones de soja, les probiotiques en général et tout particulièrement les lactobaciles appartenant à la famille de lactobacillus casei et paracasei, les bifidobacteria, les pseudomonadaceae, les antagonistes de la subtance P et plus précisément les sels de lanthanides, les dérivés de la DHEA, et leurs mélanges.

Comme agent présentant un intérêt nutritionnel ou neutraceutique, on citera à titre d'illustration des nombreuses applications possibles, les préparations céréalières, telles les germes de blé dur, le café moulu, ses extraits lyophilisés sous forme native ou décaféinée, le thé, les isoflavones de soja, et leurs mélanges.

Comme catalyseurs chimiques et biochimiques, on citera les complexes asymétriques du bore, les sels de manganèse, les porphyrines, associées au fer oxydé, les protéases, amylase, lignocellulases, et leurs mélanges.

Comme teintures on citera l'indigo, l'indigo-carmine, la tartrazine, et leurs mélanges.

Les couches de produits comprennent de préférence un support pouvant comprendre tout composé utile à l'application envisagée de la tablette: Par exemple, les couches de produit peuvent comprendre des agents de blanchiment ou encore des détergents pour une application comme compositions de lavage du linge ou de la vaisselle. Alternativement, les couches de produits peuvent comprendre des agents fertilisants pour une application en tant qu'engrais. Alternativement encore, les couches de produits peuvent comprendre des agents pesticides pour la protection des cultures. Dans une forme de réalisation de l'invention, ces couches de produits peuvent comprendre un agent libérateur de chlore pour une application de la tablette en vue de la désinfection de l'eau.

Les couches de produit peuvent comprendre en outre un ou plusieurs additifs de performance ou de procédé, choisis parmi les parfums, les colorants, les lubrifiants, les diluants, les liants, les agents anti-mousse, les agents d'aide au pastillage, et leurs mélanges.

Avantageusement, les couches de produit sont de compositions identiques.

Dans la présente demande, on entend par couche de fragmentation une couche apte à se déliter très rapidement, en particulier apte à se déliter significativement plus rapidement que les couches de produit adjacentes : en particulier, une telle couche présente un délitement significativement plus rapide que les couches de produit : par une première couche apte à se déliter plus rapidement ou présentant un délitement plus rapide qu'une deuxième couche, on entend au sens de la présente demande, que la première couche, prise seule, en l'absence de tout obstacle à son délitement naturel et spontané comme par exemple une couche de revêtement barrière additionnelle, plongée dans un liquide déterminé, comme par exemple de l'eau, se délite en un temps significativement plus court que la deuxième couche, plongée dans le même liquide dans les mêmes conditions. Par exemple, chaque couche de fragmentation est apte à se déliter au moins deux fois plus vite que chaque couche de produit adjacente. En particulier, chaque couche de fragmentation possède un agent d'éclatement à un taux apte à conférer à ladite couche de fragmentation un délitement plus rapide que celui des couches de produit. Chaque couche de fragmentation est de préférence prise en sandwich entre deux couches de produit identiques.

On entend plus particulièrement par « agent d'éclatement », au sens de la présente demande, tout ingrédient ou composition d'ingrédients qui, au contact d'un solvant de délitement, par exemple de l'eau, de la tablette :
- soit présente une augmentation de la taille moyenne des particules qui le composent d'au moins 25%,
- soit donne lieu à une dissolution des particules qui le composent à une vitesse supérieure à 0,5g/minute à température ambiante.

Selon la tablette de l'invention, la teneur en agent d'éclatement dans chaque couche de fragmentation est supérieure ou égale à 25% en poids, par rapport au poids de ladite couche de fragmentation. Un tel taux permet d'obtenir un délitement optimal de la tablette selon l'invention et en particulier un temps de délitement significativement inférieur à celui obtenu pour une tablette monocouche comprenant le même taux global, rapporté à l'ensemble de la tablette, d'agent d'éclatement, mais exempte de couche de fragmentation.

Dans une forme de réalisation de l'invention, la teneur en agent d'éclatement dans chaque couche de fragmentation est supérieure ou égale à 30% en poids, par rapport au poids de la dite couche de fragmentation. De préférence encore, cette teneur est inférieure ou égale à 70% en poids, par rapport au poids de la dite couche de fragmentation.

Dans une forme préférée de réalisation de l'invention, l'agent d'éclatement est choisi parmi les amidons, modifiés ou non, les dérivés d'amidon, les celluloses, modifiées ou non, les dérivés de cellulose, les polyacrylates réticulés, les polyvinylpyrrolidones réticulés, les polysaccharides, les alginates tels que l'acide alginique et l'alginate de sodium, les dérivés de silicate d'aluminium, la silice et/ou les gommes et dérivés, et leurs mélanges.

Les amidons, modifiés ou non, et les dérivés d'amidon peuvent être choisis parmi l'amidon de maïs, l'amidon de riz, l'amidon de blé, l'amidon de tapioca, l'amidon pré-gélatinisé, le carboxyméthylamidon sodique, et leurs mélanges. Comme agent d'éclatement convenant à la présente invention, on peut citer l'amidon de maïs, vendu sous la dénomination commerciale « STARX 1500 » par la société Staley, l'amidon pré-gélatinisé vendu sous la dénomination commerciale « LYCATAB® PGS » par la société Roquette Freres ou encore «STARCH 1500 » par la société Colorcon, le carboxyméthylamidon sodique vendu sous la dénomination commerciale « EXPLOTAB® » par la société Roquette Freres.

Comme agent d'éclatement convenant à la présente invention, on peut également citer le polyacrylate réticulé vendu sous la dénomination commerciale « ACUSOL® 772 » par la société Rohm and Haas.

Comme agent d'éclatement convenant à la présente invention, on peut également citer la polyvinylpyrrolidone réticulée vendue sous la dénomination commerciale « KOLLIDON® CL » par la société BASF.

Les celluloses, modifiées ou non, et les dérivés de cellulose peuvent être choisis parmi la cellulose amorphe, la cellulose microcristalline, les carboxyméthylcelluloses réticulées, l'hydroxypropylcellulose calcique faiblement substituée et leurs mélanges. Comme agent d'éclatement convenant à la présente invention, on peut ainsi citer la cellulose amorphe vendue sous la dénomination commerciale « ARBOCEL® A300 » par la société JRS, la cellulose microcristalline vendue sous la dénomination commerciale « AVICEL® PH 100 » ou encore « AVICEL® PH 102 » par la société FMC Biopolymeres, l'hydroxypropylcellulose calcique faiblement substituée vendue sous la dénomination commerciale « L-HPC » par la société Zhongbao Chemicals.

Comme agent d'éclatement convenant à la présente invention, on peut également citer les polysaccharides de soja.

Les dérivés de silicate d'aluminium peuvent être choisis parmi les complexes de magnésium colloïdal et de silicate d'aluminium, tel que le produit vendu sous la dénomination commerciale « VEEGUM® » par la société R. T. Vanderbilt, les silicates d'aluminium hydratés naturels, tels que la bentonite, l'argile ou le kaolin, et leurs mélanges.

Comme agent d'éclatement convenant à la présente invention, on peut également citer la silice hydrophile hautement dispersée vendue sous la dénomination commerciale « AEROSIL® 200 » par la société Degussa.

Comme gomme convenant comme agent d'éclatement de la présente invention, on peut citer la gomme guar.

Dans une forme de réalisation de l'invention, l'agent d'éclatement est la cellulose microcristalline.

Dans une autre forme de réalisation de l'invention, l'agent d'éclatement est la cellulose amorphe.

La ou les couches de fragmentation peuvent comprendre en outre au moins un agent effervescent. Dans une forme de réalisation de l'invention, au moins une couche de fragmentation comprend au moins un agent effervescent.

Dans une autre forme de réalisation de l'invention, toutes les couches de fragmentation comprennent au moins un agent effervescent.

La demanderesse a en effet constaté que si la performance d'un système effervescent comme agent accélérateur de délitement était limitée, voire nulle ou contraire, lorsqu'elle est mise en oeuvre de façon homogène dans un compact, la libération du gaz dans la couche de fragmentation, si elle est dosée correctement, induit une séparation physique plus efficace des couches de produit, accélérant ainsi la vitesse de délitement globale.

De préférence, l'agent effervescent est une association effervescente d'un polyacide organique hydrosoluble et d'une base faible. Dans ce cas, le polyacide organique hydrosoluble est choisi de préférence parmi l'acide adipique, l'acide citrique, l'acide malique, l'acide tartrique, l'acide malonique, l'acide fumarique, l'acide maléique, l'acide succinique et leurs mélanges. Ces acides peuvent être mis en oeuvre sous leur forme anhydre ou hydratée.

La base faible peut être choisie parmi le carbonate ou le bicarbonate de sodium, le carbonate ou le bicarbonate de potassium, le carbonate ou le bicarbonate de magnésium et leurs mélanges. De préférence, la base faible est le bicarbonate de sodium.

De préférence, l'agent effervescent comprend une combinaison d'acide adipique et optionnellement d'acide citrique anhydre, et de bicarbonate de sodium.

Dans une forme de réalisation de l'invention, l'agent effervescent est présent dans au moins une couche de fragmentation à une teneur allant de 10 à 50% en poids, de préférence de 20 à 40% en poids, par rapport au poids de la couche de fragmentation.

La ou les couches de fragmentation peuvent comprendre en outre au moins un sel soluble. Selon la présente invention, au moins une couche de fragmentation comprend au moins un sel soluble anhydre ou plus ou moins hydraté.

Dans une autre forme de réalisation de l'invention, toutes les couches de fragmentation comprennent au moins un sel soluble.

Le sel soluble peut être choisi parmi l'acétate de sodium, l'acétate de potassium, le sulfate de sodium, le chlorure de sodium et leurs mélanges.

De préférence, le sel soluble est le chlorure de sodium.

Dans une forme de réalisation de l'invention, le sel soluble est présent dans au moins une couche de fragmentation à une teneur allant de 30 à 80% en poids, par rapport au poids de la couche de fragmentation.

La ou les couches de fragmentation peuvent comprendre en outre un ou plusieurs additifs choisis parmi les parfums, les colorants, les lubrifiants, les diluants, les liants, les agents anti-mousse, les agents d'aide au pastillage, et leurs mélanges. La ou les couches de fragmentation peuvent également comprendre un ou plusieurs agents actifs choisis parmi les agents détergents, les agents blanchissants, les engrais et leurs mélanges.

Selon la présente invention, le taux du poids total de l'ensemble des couches de fragmentation rapporté au poids total de l'ensemble des couches de produit est au plus égal à 33%. De préférence, ce taux est inférieur ou égal à 25%, et de préférence encore inférieur ou égal à 15%.

Selon la présente invention, l'agent d'éclatement est présent dans la tablette à une teneur inférieure ou égale à 15% en poids, par rapport au poids total de la tablette. De préférence, cette teneur va de 0,01 à 4%, en poids, par rapport au poids total de la tablette. Ainsi, la tablette selon l'invention présente un délitement optimal et un temps de délitement particulièrement court, tout en présentant un taux global d'agent d'éclatement, rapporté au poids de la tablette, parfaitement raisonnable et respectueux de l'environnement. Grâce à la tablette selon l'invention, la consommation des matières premières que représentent les agents d'éclatement, et leur rejet dans la nature, sont limités.

Dans une forme de réalisation de l'invention, chaque couche de produit comprend moins de 4% en poids d'agent d'éclatement, par rapport au poids de la couche de produit. Ainsi, les couches de produit présentent un délitement très lent par rapport à celui de la ou des couches de fragmentation. De préférence, lesdites couches de produit sont exemptes d'agent d'éclatement.

De préférence, les deux couches externes de la tablette selon l'invention sont des couches de produit.

Dans une forme de réalisation de l'invention, la tablette peut comprendre des couches supplémentaires, de natures différentes des couches de produit ou des couches de fragmentation décrites ci-dessus.

La tablette selon l'invention peut être préparée selon des procédés de pastillage multicouches connus, comme par exemple la compression directe. Les différents composants de chaque couche sont généralement fournis sous forme de poudres. On prépare la composition de chaque couche par simple mélange de ces composants dans un mélangeur. Chaque couche est ensuite compactée dans une pastilleuse par application d'une force de compression. Les couches de fragmentation peuvent être réalisées avec ou sans précompression en fonction des poudres utilisées comme matières premières de départ et en fonction du taux de compaction souhaité pour ces couches. De préférence, on appliquera une faible précompression afin d'obtenir des couches de fragmentation uniformes et une tablette solide finale ne présentant pas de manque d'adhésion entre les différentes couches la composant.

La tablette selon l'invention est particulièrement utile dans le cas où la formule de la tablette a une nature chimique très différente du milieu d'utilisation, en particulier du liquide dans lequel la tablette doit se déliter. L'introduction d'agent d'éclatement à un taux élevé dans une couche de fragmentation relativement mince permet une séparation rapide des couches de produit et améliore le temps global de délitement de l'ensemble de la tablette.

### EXEMPLE 1 :

On prépare la composition suivante 1A, adaptée pour une application en détergence, par exemple pour un lave-vaisselle (les quantités sont données en pourcentage de poids, rapporté au poids total de la composition) :

### Composition 1A :

- tripolyphosphate de sodium vendu sous la dénomination commerciale « STPP » par la société PRAYON 40 %
- métasilicate de sodium vendu par la société SILMACO 51,5%
- polyacrylate vendu sous la dénomination commerciale « ACCUSOL 771 » par la société Rohm and Haas 2 %
- ester d'alcool gras ethoxylé, vendu sous la dénomination commerciale « genapol EP2544 » par la société Clariant 1 %
- stéarate de magnésium commercialisé par la société QUIMDIS 0,5%
- dichloroisocyanurate de sodium (DCCNa) vendu sous la dénomination commerciale « ACL60 » par la société Oxydental Chemical 5 %

Les composés ci-dessus sont fournis sous forme de poudre et la composition est préparée par simple mélange de ces composés.

Une tablette de 3 g de composition 1A, appelée ci-après tablette 1A, préparée par compactage classique de la composition 1A sur une pastilleuse de type « TITANT AG3 », commercialisée par la société LINOTECH à une force de compression 120 kN, présente un temps de délitement en milieu aqueux agité à 50°C de plus de 8 minutes dans 800 ml d'eau.

On prépare par simple mélange des composés la composition 1 B suivante (les quantités sont données en pourcentage de poids, rapporté au poids total de la composition) :

### Composition 1 B :

- cellulose microcristalline vendue sous la dénomination commerciale « Arbocel® A300 » par la société JRS 30 %
- chlorure de sodium vendu par la société Quadrimex 70 %

A partir des deux compositions 1A et 1 B ci-dessus, on prépare deux tablettes, une tablette 1C selon l'invention, et une tablette 1D comparative.
- La tablette 1C : on prépare la tablette 1C en compactant de façon classique, sur une pastilleuse de type « TITANT AG3 », commercialisée par la société LINOTECH à une force de compression 120 kN, une première couche de produit, de composition 1A, puis une couche de fragmentation, de composition 1 B, et enfin une deuxième couche de produit, de composition 1A. La tablette 1C est donc une tablette à trois couches, deux couches de produit séparées par une couche de fragmentation. Chaque couche de produit pèse 1,5 g et la couche de fragmentation pèse 0,3 g.

Ainsi, dans la tablette 1C :
- le taux d'agent d'éclatement (cellulose) est de 30% en poids, par rapport au poids de la couche de fragmentation,
- le taux global d'agent d'éclatement est de 2,7% en poids par rapport au poids total de la tablette,
- le taux du poids de la couche de fragmentation rapporté au poids total des deux couches de produit est de 10%.
- La tablette 1 D : on prépare la tablette 1 D en mélangeant 0,3 g de composition 1 B avec 2,7 g de composition 1A et on compacte le tout de façon classique sur une pastilleuse de type « TITANT AG3 », commercialisée par la société LINOTECH à une force de compression 120 kN.

La tablette 1 D est donc une tablette monocouche, de composition suivante (les quantités sont données en pourcentage de poids, rapporté au poids total de la tablette) :

### Tablette 1D :

- tripolyphosphate de sodium vendu sous la dénomination commerciale « STPP » par la société PRAYON 36,2 %
- métasilicate de sodium vendu par la société SILMACO 45,3%
- polyacrylate vendu sous la dénomination commerciale « ACCUSOL 771 » par la société Rohm and Haas 2 %
- ester d'alcool gras ethoxylé vendu sous la dénomination commerciale « genapol EP2544 » par la société Clariant 1 %
- stéarate de magnésium commercialisé par la société QUIMDIS 0,5 %
- dichloroisocyanurate de sodium (DCCNa) commercialisé sous la dénomination commerciale« ACL60 » par la société Oxydental Chemical 5%
- cellulose microcristalline 3 %
- chlorure de sodium vendu par la société Quadrimex 7%

La tablette 1D pèse 3 g. Le taux global d'agent d'éclatement dans la tablette 1 D est de 3% en poids par rapport au poids total de la tablette.

Le délitement et la résistance à la rupture des trois tablettes 1A, 1C et 1 D sont mesurés.

Le délitement est mesuré suivant la méthode suivante : une tablette est placée dans un bécher d'un litre contenant 800 mL d'eau à 50°C sous agitation. Le temps que met la tablette à se déliter est mesuré.

La dureté est mesurée suivant la méthode suivante : on mesure, à l'aide du duromètre « Schleuniger 8M», commercialisé par la société Pharmatron, la force nécessaire à appliquer pour briser la tablette, encore appelée résistance à la rupture (en Newton), selon le test défini dans la Pharmacopée Européenne dans la monographie 2.9.8.

Les délitements sont comparés pour des résistances à la rupture qui sont du même ordre de grandeur (+/- 20N) c'est-à-dire pour des compacts de tenue similaire. Dans le présent exemple, les résistances à la rupture des trois tablettes sont très proches ; les délitements des trois tablettes peuvent donc être comparés.

Les résultats sont récapitulés dans le tableau 1 ci-dessous.

**Tableau I :**

| Tablette | Temps de délitement (en min) | Résistance à la rupture (en N) |
|---|---|---|
| Tablette 1A | 9 | 95 |
| Tablette 1C | 5 | 98 |
| Tablette 1D | 9 | 78 |

La tablette 1C selon l'invention, comprenant une couche de fragmentation regroupant l'ensemble de l'agent d'éclatement de la tablette, se désintègre presque deux fois plus rapidement que les tablettes 1A et 1 D. En particulier, elle se désintègre beaucoup plus rapidement que la tablette 1 D qui comprend pourtant un taux global d'agent d'éclatement (3%) par rapport au poids de la tablette supérieur.

Cet exemple montre l'avantage apporté par la présente invention.

Par ailleurs, on a mesuré les temps de délitement respectifs de la couche de fragmentation et d'une couche de produit de la tablette 1C, prises seules sans obstacles à leur délitement naturel et spontané, dans de l'eau et à température ambiante (environ 20°C) : le temps de délitement de la couche de fragmentation est de : 1,5 minutes. Le temps de délitement d'une couche de produit est : 5 minutes. Ainsi, dans la tablette 1C selon l'invention, la couche de fragmentation présente un délitement significativement plus rapide, par exemple au moins deux fois plus rapide et même au moins trois plus rapide, que celui des couches de produit.

### EXEMPLE 2:

On prépare la composition suivante 2A, adaptée pour une application en détergence (les quantités sont données en pourcentage de poids, rapporté au poids total de la composition) :

### Composition 2A :

- dichloroisocyanurate de sodium dihydraté vendu par la société Oxychem sous la dénomination commerciale « ACL56 » 76%
- lauryl sulfate de sodium vendu sous la dénomination commerciale « Texapon® K12P » par la société Cognis 2 %
- bicarbonate de sodium vendu par la société Solvay 9%
- Acide adipique vendu par la société BASF 13 %

La composition est préparée par simple mélange des composés fournis sous forme de poudres.

Une telle composition peut être utilisée pour compacter des tablettes pour désinfecter, notamment les sols et surfaces. Selon les applications (particulier ou collectivité), les quantités requises ne sont pas les mêmes. Un particulier utilisera des tablettes de l'ordre de 3 g alors que les collectivités préféreront des tablettes de masse plus élevée, par exemple de 6,3 g.

On a préparé par compactage classique sur une pastilleuse de type « TITANT AG3 », commercialisée par la société LINOTECH à une force de compression 130 kN une tablette 2A et une tablette 2A', respectivement de 3 g et de 6,3 g, toutes deux de composition 2A. On a ensuite mesuré les temps de délitement respectifs de ces tablettes selon la méthode suivante : chaque tablette est délitée dans un bécher d'un Litre contenant 800mL d'eau à 20°C. Les résultats obtenus pour le temps de délitement, en minutes, sont les suivants :
Tablette 2A : 18 min
Tablette 2A' : 30 min.

On prépare par simple mélange des composés fournis sous forme de poudres la composition 2B suivante (les quantités sont données en pourcentage de poids, rapporté au poids total de la composition) :

### Composition 2B :

- cellulose microcristalline vendue sous la dénomination commerciale « Arbocel® A300 » par la société JRS 41 %
- acide adipique vendu par la société BASF 11%
- bicarbonate de sodium vendu par la société Solvay 16 %
- chlorure de sodium vendu par la société Quadrimex 32%

A partir des deux compositions 2A et 2B ci-dessus, on prépare deux tablettes selon l'invention, les tablettes 2C et 2C' :
Tablette 2C : on prépare la tablette 2C en compactant de façon classique sur une pastilleuse de type « TITANT AG3 », commercialisée par la société LINOTECH à une force de compression 130 kN, une première couche de produit, de composition 2A, puis une couche de fragmentation, de composition 2B, et enfin une deuxième couche de produit, de composition 2A. La tablette 2C est donc une tablette à trois couches, deux couches de produit séparées par une couche de fragmentation, la couche de fragmentation représentant 22% en poids, par rapport au poids des deux couches de produit. La tablette 2C pèse 3 g.
Tablette 2C' : on prépare la tablette 2C' en compactant de façon classique sur une pastilleuse de type « TITANT AG3 », commercialisée par la société LINOTECH à une force de compression 130 kN, quatre couches de produit, de composition 2A, et trois couches de fragmentation, de composition 2B, chaque couche de fragmentation séparant deux couches de produit. La tablette 2C' est donc une tablette à sept couches, les couches externes étant des couches de produit. L'ensemble des couches de fragmentation représente 22% en poids, par rapport au poids de l'ensemble des couches de produit. La tablette 2C' pèse 6,3 g.

Les temps de délitement sont mesurés selon la méthode décrite pour les compositions 2A et 2A'. Les résultats obtenus pour le temps de délitement, en min, sont les suivants :
Tablette 2C : 5,5 min
Tablette 2C' : 6 min.

La fragmentation des tablettes 2A et 2A', par l'introduction d'une ou plusieurs couches séparatrices, améliore considérablement le temps de délitement.

L'augmentation du nombre de couches séparatrices, ou de fragmentation, permet d'obtenir un même temps de délitement pour deux masses de compositions solides à déliter différentes, tout en diminuant significativement la durée totale de délitement même pour la tablette la plus petite. Cet exemple montre l'avantage apporté par la présente invention.

### EXEMPLE 3 :

On cherche à amener en solution 3,95 grammes d'une tablette de composition analogue à celle de la composition 1A de l'Exemple 1.

On évalue la performance d'une composition d'aide au délitement, appelée Composition 3B, dont la masse totale est de 0,05 g et dont la composition est donnée ci-dessous. La Composition 3B est soit mélangée à la composition 1A pour donner une tablette monocouche ci-après appelée Tablette 3D, soit intercalée comme couche de fragmentation au sein d'une tablette à trois couches comprenant deux couches de produit de composition 1A et une couche de fragmentation de composition 3B, appelée Tablette 3C.

Composition 3B (les quantités sont données en pourcentage de poids, rapporté au poids total de la composition) :
- cellulose amorphe vendue sous la dénomination commerciale « ARBOCEL A300 » par la société JRS 20 %
- chlorure de sodium 66,7 %
- acide adipique 5,3 %
- bicarbonate de sodium 8%

Ramené aux tablettes 3C et 3D, cette composition apporte 0,25% d'agent d'éclatement pour une masse finale de 4g.

Les tablettes 3C et 3D sont préparées par compactage classique sur une pastilleuse de type « TITANT AG3 », commercialisée par la société LINOTECH à une force de compression 130 kN pour la tablette 3C, à une force de compression 130 kN respectivement pour la tablette 3D.

Le temps de délitement est mesuré pour la tablette 3C et pour la tablette 3D selon la méthode suivante : la tablette est introduite dans un panier puis celui-ci est introduit dans un bécher de 1 L rempli d'eau à 50°C sous agitation magnétique.

Pour chaque type de tablette, trois essais sont réalisés.

Les résultats obtenus sont rassemblés dans les tableaux II et III suivants :

**Tablette 3D : Tableau II**

| | Essai 1 | Essai 2 | Essai 3 |
|---|---|---|---|
| Densité vraie en g/cm³ mesurée au pycnomètre | 2,0503 | 1,9991 | 2,0229 |
| Temps de délitement en min | 55 min | 55 min | 60 min |
| Masse en g | 4,0178 | 3,9988 | 4,1639 |
| Volume en cm³ | 2,363 | 2,350 | 2,456 |
| Masse/volume en g/cm³ | 1,700 | 1,702 | 1,695 |
| (Masse/volume)/Densité vraie | 0,828 | 0,851 | 0,824 |

**Tablette 3C : Tableau III**

| | Essai 1 | Essai 2 | Essai 3 |
|---|---|---|---|
| Densité vraie en g/cm³ mesurée au pycnomètre | 2,0119 | 2,0095 | 2,0451 |
| Temps de délitement en min | 55 min | 55 min | 30 min |
| Masse en g | 4,2226 | 4,1036 | 4,2650 |
| Volume en cm³ | 2,482 | 2,435 | 2,547 |
| Masse/volume en g/cm³ | 1,701 | 1,685 | 1,674 |
| (Masse/volume)/Densité vraie | 0,845 | 0,838 | 0,818 |

On observe que la performance du système fragmentant est particulièrement décevante. En effet:
1. deux tablettes tricouches sur trois ont un temps de délitement analogue à celui des tablettes monocouches
2. Une seule tablette a fourni une fragmentation performante.

Les tablettes ayant été obtenues par un procédé de compression similaire (densités obtenues quasi-identiques), on en conclut qu'un taux de 20% en poids d'agent d'éclatement par rapport au poids de la couche de fragmentation est insuffisant pour permettre une bonne fragmentation.

On s'intéresse alors à une composition d'aide au délitement à 30% d'agent d'éclatement, soit la composition 4B.

Composition 4B (les quantités sont données en pourcentage de poids, rapporté au poids total de la composition) :
- cellulose amorphe vendue sous la dénomination commerciale « ARBOCEL A300 » par la société JRS 30 %
- chlorure de sodium 50 %
- acide adipique 8 %
- bicarbonate de sodium 12 %

Ramené aux tablettes 4C et 4D, cette composition apporte environ 0,37% d'agent d'éclatement pour une masse finale de 4g.

Les tablettes 4C et 4D sont préparées par compactage classique sur une pastilleuse de type « TITANT AG3 », commercialisée par la société LINOTECH à une force de compression 130 kN pour la tablette 4C, à une force de compression 130 kN respectivement pour la tablette 4D.

Le temps de délitement est mesuré selon la même méthode que pour les tablettes 3C et 3D.

Les résultats obtenus sont rassemblés dans les tableaux IV et V suivants :

**Tablette 4D : Tableau IV**

| | Essai 1 | Essai 2 | Essai 3 |
|---|---|---|---|
| Densité vraie en g/cm³ mesurée au pycnomètre | 2,0099 | 2,0325 | 2,0098 |
| Temps de délitement en min | 55 min | 54 min 45 s | 60 min |
| Masse en g | 4,1802 | 4,1113 | 3,9804 |
| Volume en cm³ | 2,37 | 2,44 | 2,35 |
| Masse/volume en g/cm³ | 1,7637 | 1,6849 | 1,6938 |
| (Masse/volume)/Densité vraie | 0,877 | 0,829 | 0,843 |

**Tablette 4C : Tableau V**

| | Essai 1 | Essai 2 | Essai 3 |
|---|---|---|---|
| Densité vraie en g/cm³ mesurée au pycnomètre | 2,0037 | 2,0180 | 2,0128 |
| Temps de délitement en min | 32 min 40 s | 37 min | 26 min |
| Masse en g | 3,5935 | 4,1602 | 4,0122 |
| Volume en cm³ | 2,148 | 2,564 | 2,418 |
| Masse/volume en g/cm³ | 1,673 | 1,622 | 1,659 |
| (Masse/volume)/Densité vraie | 0,835 | 0,804 | 0,824 |

On observe que la composition 4B, qui comprend 30% en poids d'agent d'éclatement par rapport au poids de la composition, constitue une couche de fragmentation significativement efficace puisque les trois essais de tablettes 4C possédant cette couche montrent un temps de délitement raccourci, environ d'un facteur 2. En revanche, la composition 3B, qui ne comprend que 20% en poids d'agent d'éclatement par rapport au poids de la composition ne permet pas d'obtenir une amélioration significative du temps de délitement lorsqu'on l'utilise comme couche de fragmentation pour passer d'une tablette monocouche 3D à une tablette à trois couches 3C.

## Revendications

1. Tablette solide multicouche, destinée à se déliter dans un liquide pour libérer dans ledit liquide un agent actif, comprenant au moins n couches de fragmentation, n étant un nombre entier au moins égal à 1, et au moins (n + 1) couches de produit, lesdites (n + 1) couches de produit étant de compositions identiques, chaque couche de produit comprenant au moins ledit agent actif, chaque couche de fragmentation étant disposée entre deux couches de produit et présentant un délitement significativement plus rapide que celui desdites couches de produit, chaque couche de fragmentation comprend au moins un agent d'éclatement, ledit agent d'éclatement étant présent dans ladite couche de fragmentation à une teneur supérieure ou égale à 25% en poids, par rapport au poids de la dite couche de fragmentation,
**caractérisée en ce que** :
le taux du poids total de l'ensemble des couches de fragmentation rapporté au poids total de l'ensemble des couches de produit est au plus égal à 33%,
l'agent d'éclatement est présent dans la tablette à une teneur inférieure ou égale à 15% en poids, par rapport au poids total de la tablette, et
au moins une couche de fragmentation comprend au moins un sel soluble.

2. Tablette selon la revendication 1, **caractérisée en ce que** ladite teneur est supérieure ou égale à 30% en poids, par rapport au poids de la dite couche de fragmentation.

3. Tablette selon la revendication 1 ou 2, **caractérisée en ce que** ladite teneur est inférieure ou égale à 70% en poids, par rapport au poids de la dite couche de fragmentation.

4. Tablette selon l'une quelconque des revendications 1 à 3, **caractérisée en ce que** l'agent d'éclatement est choisi parmi les amidons, modifiés ou non, les dérivés d'amidon, les celluloses, modifiées ou non, les dérivés de cellulose, les polyacrylates réticulés, les polyvinylpyrrolidones réticulés, les polysaccharides, les alginates tels que l'acide alginique et l'alginate de sodium, les dérivés de silicate d'aluminium, la silice et/ou les gommes et dérivés, et leurs mélanges.

5. Tablette selon l'une quelconque des revendications 1 à 4, **caractérisée en ce que** l'agent d'éclatement est présent dans la tablette à une teneur allant de 0,01 à 4% en poids, par rapport au poids total de la tablette.

6. Tablette selon l'une quelconque des revendications précédentes, **caractérisée en ce que** chaque couche de produit comprend moins de 4% en poids, d'agent d'éclatement, par rapport au poids de la couche de produit.

7. Tablette selon l'une quelconque des revendications 1 à 5, caractérisée en ce lesdites couches de produit sont exemptes d'agent d'éclatement.

8. Tablette selon l'une quelconque des revendications précédentes **caractérisée en ce qu'**au moins une couche de fragmentation comprend au moins un agent effervescent.

9. Tablette selon la revendication précédente, **caractérisée en ce que** l'agent effervescent est présent dans la couche de fragmentation à une teneur allant de 10 à 50% en poids, de préférence de 20 à 40% en poids, par rapport au poids de la couche de fragmentation.

10. Tablette selon l'une quelconque des revendications précédentes, **caractérisée en ce que** le sel soluble est présent dans la couche de fragmentation à une teneur allant de 30 à 80% en poids, par rapport au poids de la couche de fragmentation.

## Patentansprüche

1. Feste mehrschichtige Tablette, die dazu bestimmt ist, sich in einer Flüssigkeit aufzulösen, um in dieser Flüssigkeit einen Wirkstoff freizugeben, die mindestens n Zerfallsschichten umfasst, wobei n eine ganze Zahl mindestens gleich 1 ist, und mindestens (n + 1) Produktschichten, wobei die (n + 1) Produktschichten identisch zusammengesetzt sind, wobei jede Produktschicht mindestens den Wirkstoff umfasst, wobei jede Zerfallsschicht zwischen zwei Produktschichten angeordnet ist und sich signifikant schneller auflöst als die Produktschichten, wobei jede Zerfallsschicht mindestens ein Berstmittel umfasst, wobei das Berstmittel in der Zerfallsschicht in einem Gehalt von über oder gleich 25 Gew.-% im Verhältnis zum Gewicht der Zerfallsschicht vorhanden ist,
**dadurch gekennzeichnet, dass**:
der Anteil des Gesamtgewichts aller aufgebrachten Zerfallsschichten im Verhältnis zum Gesamtgewicht aller Produktschichten höchstens gleich 33 % beträgt,
das Berstmittel in der Tablette in einem Gehalt von unter oder gleich 15 Gew.-% im Verhältnis zum Gesamtgewicht der Tablette vorhanden ist, und
mindestens eine Zerfallsschicht mindestens ein lösliches Salz umfasst.

2. Tablette nach Anspruch 1, **dadurch gekennzeichnet, dass** der Gehalt im Verhältnis zum Gewicht der Zerfallsschicht über oder gleich 30 Gew.-% beträgt.

3. Tablette nach Anspruch 1 oder 2, **dadurch gekennzeichnet, dass** der Gehalt im Verhältnis zum Gewicht der Zerfallsschicht unter oder gleich 70 Gew.-% beträgt.

4. Tablette nach einem der Ansprüche 1 bis 3, **dadurch gekennzeichnet, dass** das Berstmittel aus den modifizierten oder nicht modifizierten Stärken, den Stärkederivaten, den modifizierten oder nicht modifizierten Zellulosen, den Zellulosederivaten, den vernetzten Polyacrylaten, den vernetzten Polyvinylpyrrolidonen, den Polysacchariden, den Alginaten wie der Alginsäure und dem Natriumalginat, den Aluminiumsilikatderivaten, der Kieselsäure und/oder den Gummi und Derivaten und ihren Gemischen ausgewählt ist.

5. Tablette nach einem der Ansprüche 1 bis 4, **dadurch gekennzeichnet, dass** das Berstmittel in der Tablette im Verhältnis zum Gesamtgewicht der Tablette in einem Gehalt von 0,01 bis 4 Gew.-% vorhanden ist.

6. Tablette nach einem der vorangehenden Ansprüche, **dadurch gekennzeichnet, dass** jede Produktschicht im Verhältnis zum Gewicht der Produktschicht weniger als 4 Gew.-% Berstmittel umfasst.

7. Tablette nach einem der Ansprüche 1 bis 5, **dadurch gekennzeichnet, dass** die Produktschichten kein Berstmittel enthalten.

8. Tablette nach einem der vorangehenden Ansprüche, **dadurch gekennzeichnet, dass** mindestens eine Zerfallsschicht mindestens ein Brausemittel umfasst.

9. Tablette nach vorangehendem Anspruch, **dadurch gekennzeichnet, dass** das Brausemittel in der Zerfallsschicht im Verhältnis zum Gewicht der Zerfallsschicht in einem Gehalt von 10 bis 50 Gew.-%, vorzugsweise von 20 bis 40 Gew.-%, vorhanden ist.

10. Tablette nach einem der vorangehenden Ansprüche, **dadurch gekennzeichnet, dass** das lösliche Salz in der Zerfallsschicht im Verhältnis zum Gewicht der Zerfallsschicht in einem Gehalt von 30 bis 80 Gew.-% vorhanden ist.

## Claims

1. A multilayer solid tablet intended to disintegrate in a liquid to release an active agent in said liquid, comprising at least n fragmentation layers, n being an integer at least equal to 1, and at least (n+1) product layers, the said (n+1) product layers being of identical composition, each product layer comprising at least the said active agent, each fragmentation layer being arranged between two product layers and having significantly faster disintegration than the said product layers, each fragmentation layer comprises at least one burst agent the said burst agent being contained in the said fragmentation layer at a content equal to or higher than 25 % by weight relative to the weight of the said fragmentation layer,
**characterized in that**:
the percentage of the total weight of all the fragmentation layers relative to the total weight of all the product layers is no more than 33 %;
the burst agent is contained in the tablet at a content equal to or lower than 15 % by weight relative to the total weight of the tablet; and
at least one fragmentation layer comprises at least one soluble salt.

2. The tablet according to claim 1 **characterized in that** the said content is equal to or higher than 30 % by weight relative to the weight of the said fragmentation layer.

3. The tablet according to claim 1 or 2, **characterized in that** the said content is equal to or lower than 70 % by weight relative to the weight of the said fragmentation layer.

4. The tablet according to any of claims 1 to 3, **characterized in that** the burst agent is chosen from among starches whether or not modified, starch derivatives, celluloses whether or not modified, cellulose derivatives, cross-linked polyacrylates, cross-linked polyvinylpyrrolidones, polysaccharides, alginates such as alginic acid and sodium alginate, derivatives of aluminium silicate, silica and/or gums and derivatives and mixtures thereof.

5. The tablet according to any of claims 1 to 4, **characterized in that** the burst agent is contained in the tablet at a content ranging from 0.01 to 4 % by weight, relative to the total weight of the tablet.

6. The tablet according to any of the preceding claims, **characterized in that** each product layer comprises less than 4 % by weight of burst agent relative to the weight of the product layer.

7. The tablet according to any of claims 1 to 5, **characterized in that** the said product layers are devoid of burst agent.

8. The tablet according to any of the preceding claims, **characterized in that** at least one fragmentation layer comprises at least one effervescent agent.

9. The tablet according to the preceding claim, **characterized in that** the effervescent agent is contained in the fragmentation layer at a content ranging from 10 to 50 % by weight, preferably 20 to 40 % by weight relative to the weight of the fragmentation layer.

10. The tablet according to any of the preceding claims, **characterized in that** the soluble salt is contained in the fragmentation layer at a content ranging from 30 to 80 % by weight relative to the weight of the fragmentation layer.
